# EUROPEAN PATENT SPECIFICATION

(11) **EP 2 939 639 B1**
(45) Date of publication and mention of the grant of the patent: **07.09.2016**
(21) Application number: 15170437.6
(22) Date of filing: 05.11.2009
(51) Int. Cl.: A61F 2/46, A61F 2/44, A61F 2/28, A61F 2/30

(54) **SELF-PIVOTING SPINAL IMPLANT AND ASSOCIATED INSTRUMENTATION**
SELBSTDREHENDE WIRBELSÄULENIMPLANTAT UND ZUGEHÖRIGE INSTRUMENTE
IMPLANT RACHIDIEN AUTO-PIVOTANT ET INSTRUMENTATIONS ASSOCIÉES

(43) Date of publication of application: 04.11.2015
(62) Divisional of application: 09752956.4
(73) Proprietor: Synthes GmbH, 4436 Oberdorf (CH)
(72) Inventor: Lindenmann, Philippe, 4053 Basel (CH); Saidha, Sean, 4059 Basel (CH); Baudouin, Cyril, 68400 Riedisheim (FR); Fatone, Peter, Exton, MA Massachusetts 19341 (US)
(74) Representative: Klunker . Schmitt-Nilson . Hirsch

(56) References cited:
- WO-A1-2007/070751
- WO-A2-2008/036636
- US-A1- 2006 106 460
- US-A1- 2006 229 627
- US-A1- 2007 282 441
- US-A1- 2008 119 935
- US-A1- 2008 306 488

## Description

### BACKGROUND OF THE INVENTION

The unilateral transforaminal insertion of an interbody spacer for lumbar spinal fusion presents challenges to the surgeon tasked with the procedure due to the curved manipulation path that the implant must undergo once it enters the disc space. The procedure presents a further challenge of coupling the implant to the inserter instrument while allowing the implant a limited amount of rotation or articulation to follow the desired path. These challenges also present themselves to other angular unilateral approaches to the spine, in which the initial access corridor is linear yet, once the implant enters the disc space, the implant must be manipulated or articulated along a curved path. Conventional transforaminal lateral interbody fusion (TLIF) implants, for example, are inserted using a combination of a linear insertion path and a hammering of the implant into the desired position using pushers that provide the desired anterior positioning of the implant. Alternately, a stepwise straight hammering process alternating with an active turning technique is often used to manipulate the implant from the entry position to the final desired position. The conventional TLIF and other angular unilateral systems and insertion methods fail to provide implants, instrumentation, and methods that allow the implant to be easily inserted to its final desired position within the disc space.

It is therefore desired to provide a spinal implant and associated instrument and method that improves the ease with which the implant may be manipulated during insertion or once within the disc space.

### BRIEF SUMMARY OF THE INVENTION

Briefly stated, a first embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for engagement with a pivotable insertion instrument.

Another embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. Each anterior edge has a generally linear portion proximate the engagement end and a generally concave portion, and each posterior edge has a generally linear portion proximate the engagement end and a generally convex portion. The generally linear portion of the anterior edge converges with the generally linear portion of the posterior edge at the engagement end for each of the first and second main surfaces. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for engagement with a pivotable insertion instrument.

Still another embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. Each anterior edge is generally concave and each posterior edge is generally convex. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from the anterior and posterior walls and extending at least partially between the first and second main surfaces. The post includes a plurality of facets disposed around an entire periphery thereof and is configured for engagement with a pivotable insertion instrument. At least one abutment surface is disposed within the slot distally from the post. The at least one abutment surface limits rotation of the implant about the post when the post is engaged with the pivotable insertion instrument.

Yet another embodiment of the present invention comprises an intervertebral implant including an insertion end, an opposing engagement end, and first and second opposed main surfaces configured to contact respective adjacent vertebral endplates. Each of the first-and second main surfaces has an anterior edge, a posterior edge, and extends between the insertion and engagement ends. Each anterior edge is generally concave and each posterior edge is generally convex. An axial bore is formed between the anterior and posterior edges and extends between the first and second main surfaces. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends at least partially along the anterior and posterior walls. A post is positioned within the slot and extends at least partially between the first and second main surfaces. The post includes a plurality of facets and is configured for engagement with a pivotable insertion instrument. The intervertebral implant also includes a plurality of markers. At least one of the markers extends between the first and second main surfaces within one of the anterior and posterior walls. At least one other of the markers is disposed generally transverse to the at least one of the markers and extends from the insertion end toward the axial bore.

A still further embodiment of the present invention comprises a method for implanting an intervertebral implant into a disc space disposed between first and second endplates of adjacent vertebral bodies of a patient. The method includes providing an access corridor to a spinal level in need, removing at least a portion of disc material between the adjacent vertebra, and providing an interbody spacer implant. The implant includes an insertion end, an opposing engagement end, and first and second opposed main surfaces configured to contact the respective first and second vertebral endplates. Each of the first and second main surfaces has an anterior edge, a posterior edge, and extends between the insertion and engagement ends. Each anterior edge is generally concave and each posterior edge is generally convex. Each of the first and second main surfaces includes a plurality of curved parallel ridges protruding from the respective surface and extending from the insertion end to the engagement end. Each of the plurality of parallel ridges includes a plurality of teeth. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for engagement with a pivotable insertion instrument. The method also includes providing an insertion instrument. The instrument includes a proximal end, a distal end, and a longitudinal axis therebetween, and an inner member and an outer member. The inner member is movable along the longitudinal axis with respect to the outer member. The inner member has a grasping portion at the distal end. The grasping portion includes a plurality of facet surfaces configured for engagement with the plurality of the post facets. The method also includes inserting the grasping portion of the instrument into the slot of the implant such that the grasping portion surrounds the post, engaging the post of the implant with the grasping portion of the instrument such that the post is rotationally fixed with respect to the grasping portion, inserting the implant using the instrument through the access corridor until at least the insertion end is introduced into the at least partially cleared out disc space and such that the at least a portion of the ridges of the first and second main surfaces contact the first and second vertebral endplates, respectively, adjusting the instrument such that the post of the implant remains engaged with the grasping portion of the instrument but rotation of the post is permitted within the grasping portion, delivering impaction forces to the proximal end of the instrument such that the post of the implant articulates with respect to the grasping portion of the instrument and the implant is guided by vertebral rails into a desired position, releasing the post of the implant from the grasping portion of the instrument, and withdrawing the instrument through the access corridor.

Yet another embodiment of the present invention comprises a system for spine surgery at a disc space disposed between first and second endplates of adjacent vertebral bodies of a patient. The system includes an intervertebral implant including an insertion end and an opposing engagement end. First and second opposed main surfaces are configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets. A trial implant includes an insertion end and an opposing engagement end. First and second opposed main surfaces are configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets. An insertion instrument includes a proximal end, a distal end, a longitudinal axis therebetween, an inner member, and an outer member. The inner member is translatable with respect to the outer member along the longitudinal axis and has a grasping portion at the distal end. The grasping portion includes a plurality of facet surfaces engagable with the plurality of the post facets of the intervertebral implant and the trial implant. The instrument has a first configuration in which the grasping portion assumes an open configuration for allowing coupling of the instrument to the post of one of the intervertebral implant and the trial implant, a second configuration in which the instrument is securely coupled to the post of one of the intervertebral implant and the trial implant while allowing the post to rotate within the grasping portion under a given force, and a third configuration wherein the instrument is securely coupled to the post of one of the intervertebral implant and the trial implant while preventing rotation of the post with respect to the grasping portion.

### BRIEF DESCRIPTION OF THE SEVERAL VIEWS OF THE DRAWINGS

The foregoing summary, as well as the following detailed description of preferred embodiments of the instrument of the present application, will be better understood when read in conjunction with the appended drawings. For the purposes of illustrating the self-pivoting spinal implant and the associated instrumentation of the present application, there is shown in the drawings preferred embodiments. It should be understood, however, that the application is not limited to the precise arrangements and instrumentalities shown. In the drawings:
Fig. 1 is a rear perspective view of a self-pivoting TLIF implant in accordance with a first preferred embodiment of the present invention;
Fig. 2 is a front perspective view of the self-pivoting TLIF implant of Fig. 1;
Fig. 3 is a top plan view of the self-pivoting TLIF implant of Fig. 1;
Fig. 4 is a front and left side perspective view of the self-pivoting TLIF implant of Fig. 1;
Fig. 5 is a front perspective view of the self-pivoting TLIF implant of Fig. 1 and a bone growth promoting material configured for insertion into the implant;
Fig. 6 is a front perspective view of the self-pivoting TLIF implant of Fig. 1 showing a preferred arrangement of radiopaque markers;
Fig. 7 is a partial front perspective cross-sectional view of an inserter instrument in accordance with a first preferred embodiment of the present invention, the inserter instrument shown in open configuration;
Fig. 8A is a top plan view of the inserter instrument of Fig. 7;
Fig 8B is a partial front perspective view of the inserter instrument of Fig. 7 in the open configuration;
Fig. 9A is a top plan view of the inserter instrument of Fig. 7 in an initial articulation position and in a finally locked configuration;
Fig. 9B is a cross-sectional view of the inserter instrument of Fig. 7 in the initial articulation position and in the finally locked configuration;
Fig. 9C is a cross-sectional view of the inserter instrument of Fig. 7 in the initial articulation position and in a provisionally locked configuration;
Fig. 9D is a cross-sectional view of the inserter instrument of Fig. 7 in a final articulation position and in the provisionally locked configuration;
Fig. 10A is a cross-sectional view of the inserter instrument of Fig. 7 in the final articulation position and in the finally locked configuration;
Fig. 10B is a top plan view of the inserter instrument of Fig. 7 in the final articulation position and in the finally locked configuration;
Fig. 10C is a cross-sectional view of the inserter instrument of Fig. 7 in the final articulation position and in the open configuration;
Fig. 11A is a top plan view, partially broken away, of one position of the implant of Fig. 1 and the instrument of Fig. 7 with respect to a disc space, partially broken away, as the implant is inserted therein;
Fig. 11 B is a top plan view, partially broken away, of a the implant and instrument of

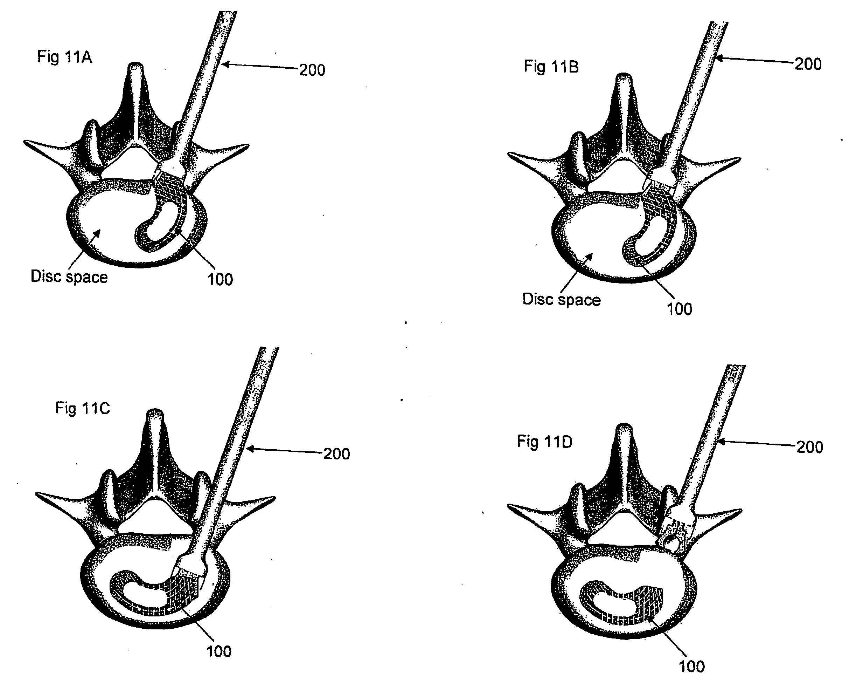

Fig. 11A in a second position;
Fig. 11C is a top plan view, partially broken away, of a the implant and instrument of Fig. 11 B in a third position;
Fig. 11D is a top plan view of a the implant and instrument of Fig. 11C in a fourth position;

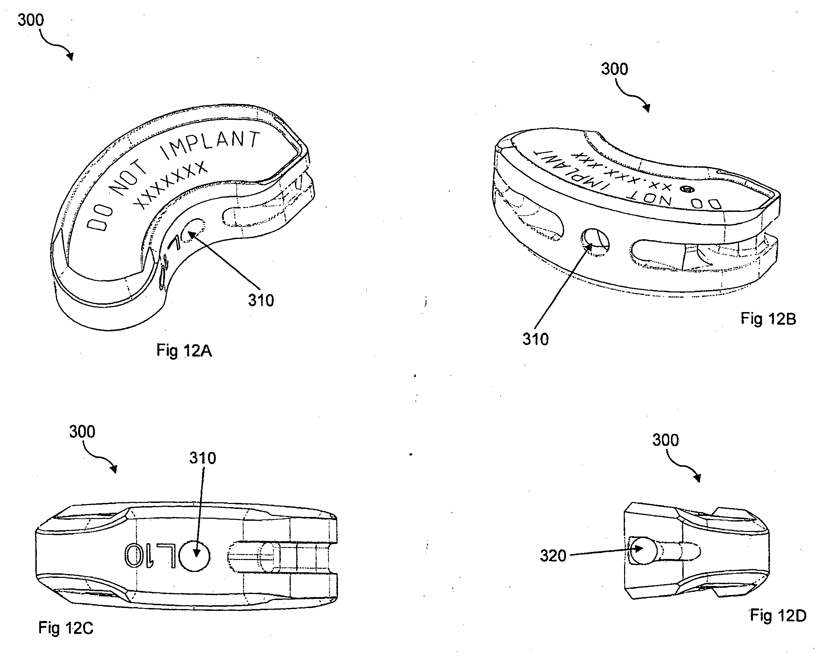

Fig. 12A is a rear perspective view of a trial implant in accordance with one embodiment of the present invention;
Fig. 12B front perspective view of the trial implant of Fig. 12A;
Fig. 12C is a rear elevational view of the trial implant of Fig. 12A; and
Fig. 12D is a left side elevational view of the trial implant of Fig. 12A.

### DETAILED DESCRIPTION OF THE INVENTION

Certain terminology is used in the following description for convenience only and is not limiting. The words "right," "left," "lower," and "upper" designate directions in the drawings to which reference is made. The words "inwardly" or "distally" and "outwardly" or "proximally" refer to directions toward and away from, respectively, the patient's body, or the geometric center of the interbody spacer implant and related parts thereof. The words, "anterior," "posterior," "superior," "inferior," and related words and/or phrases designate preferred positions and orientations in the human body to which reference is made and are not meant to be limiting. The terminology includes the above-listed words, derivatives thereof and words of similar import.

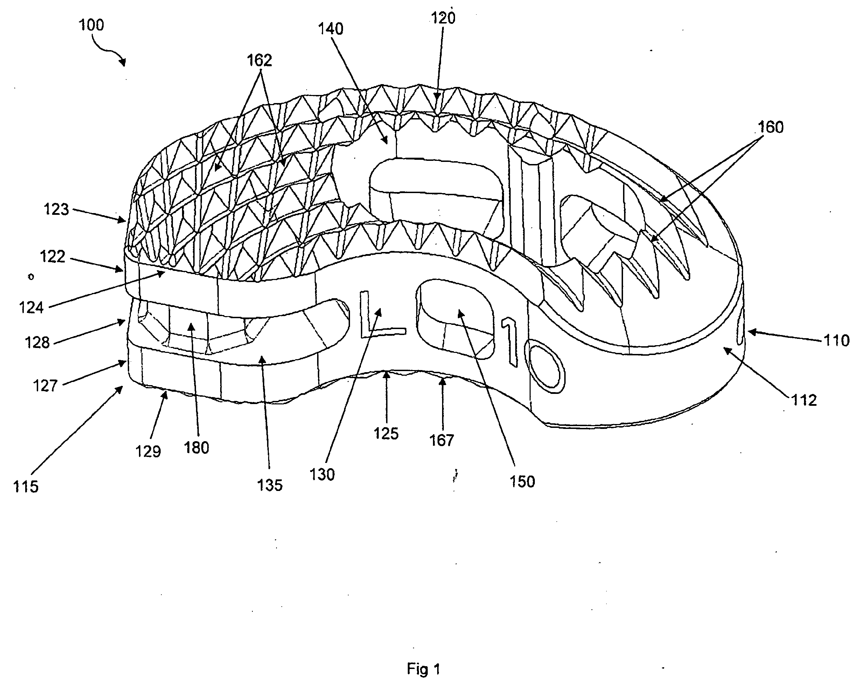

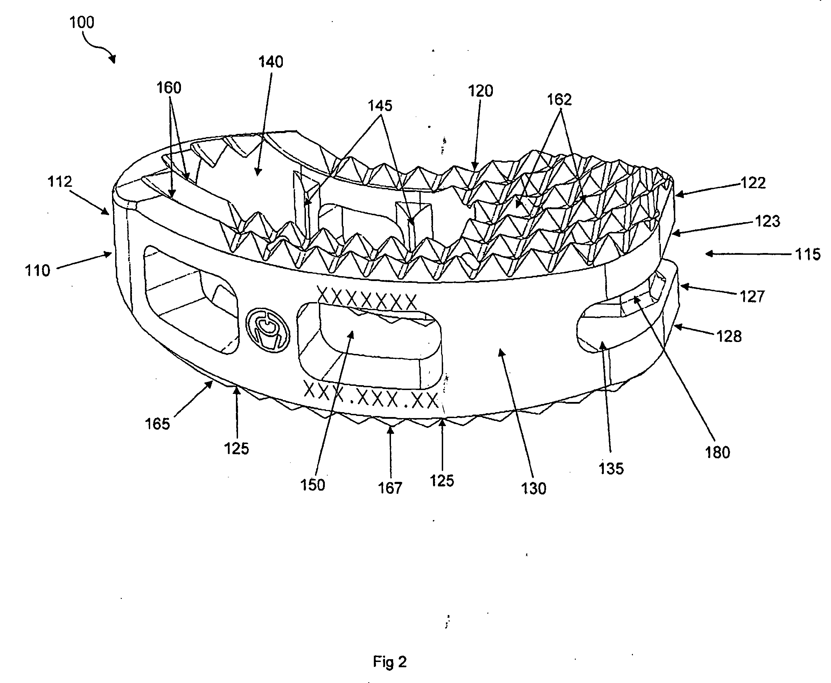

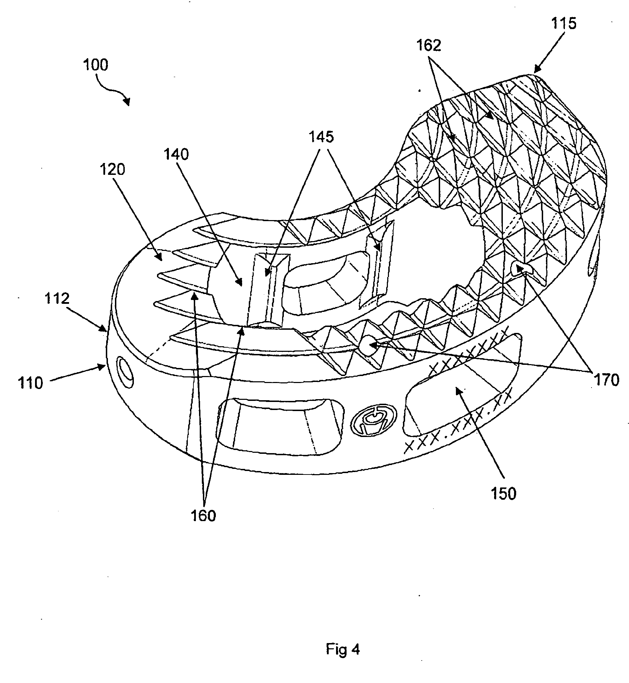

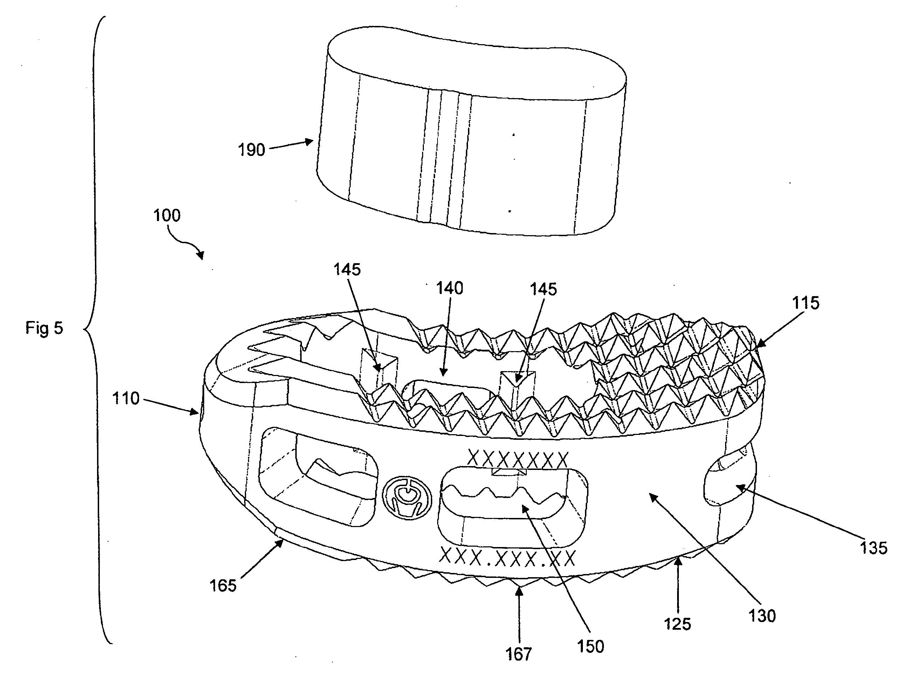

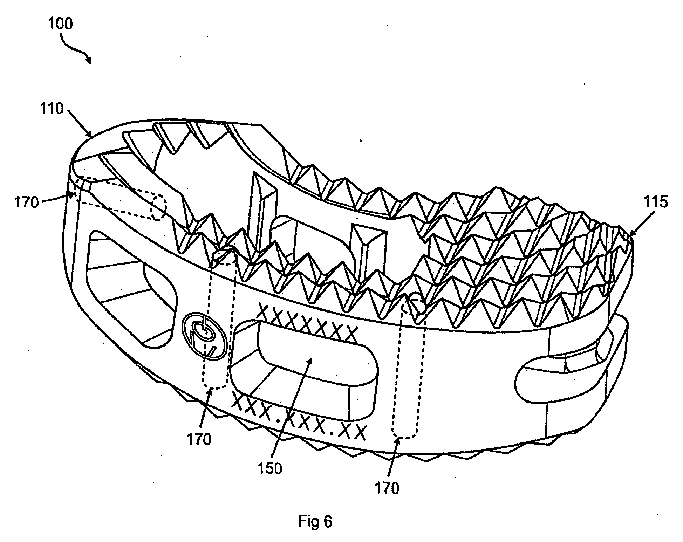

Referring to Figs. 1-6, a TLIF spacer 100 is provided that includes an insertion end 110 and an engagement end 115, the insertion end 110 preferably forming a bullet-nose 112 or having some other tapered geometry for enhancing the ease of insertion and/or for applying a distraction force to the two vertebral bodies between which the implant 100 is configured to be inserted. The implant 100 further includes a first main or superior surface 120 that is configured for contacting the inferior endplate of a superior vertebral body and a second main or inferior surface 125 that is configured for contacting the superior endplate of an inferior vertebral body. One or more walls 130 on anterior and posterior sides extend between the superior and inferior surfaces 120, 125 and enclose an axial bore 140 that extends through both the superior and inferior surfaces 120, 125. The axial bore 140 is configured to house a bone graft 190 or other fusion enhancing material.

One or more lateral windows 150 are disposed in the walls 130 and provide a visibility window for observing the fusion occurring between the vertebral bodies and enhancing the vascularization of the bone graft 190 disposed within the axial bore 140 to assist fusion, as well as to increase the volume of the axial bore 140. One or more surface features 145 are provided along interior portions of the walls 130 that form the axial bore 140 to assist in securing the bone graft 190 within the axial bore 140. The features 145 can assume the form of one or more ridges extending through the axial bore 140 along the cranial-caudal direction, grooves, or other surface texturing that enhances the friction between the bone graft 190 and the interior of the walls 130 that form the axial bore 140.

In a first preferred embodiment, the TLIF spacer 100 has a kidney bean or banana shape having a curvilinear geometry between its insertion and engagement ends 110, 115.

This shape may be accomplished by having an anterior edge of the superior and inferior surfaces 120, 125 along with the anterior wall 130 be generally concave and a posterior edge of the superior and inferior surfaces 120, 125 along with the posterior wall 130 be generally convex. However, a variety of geometries may be utilized for the implant 100, depending on the desired amount of surface contact between the endplates of the vertebral bodies and the implant 100, the number of implants 100 desired to be implanted within the disc space (e.g., one or two), the approach chosen for the surgery, the desired location of the implant within the disc space (anterior or posterior), or the like. Disposed upon the superior surface 120 adjacent the insertion end 110 are a plurality of curvilinear superior ridges 160 that are arranged parallel to one another along the curvature of the TLIF implant 100.

In a first preferred embodiment, the superior ridges 160 include two linearly sloped surfaces that meet to form an apex. As the superior ridges 160 extend along their curvilinear path away from the insertion end 110, the superior ridges 160 are interrupted to form a plurality of superior teeth 162. The superior teeth 162 are disposed at the engagement end 115 and along at least a portion of anterior and posterior sides of the axial bore 140. Similarly, disposed upon the inferior surface 125 adjacent the insertion end 110 is a plurality of curvilinear inferior ridges 165 that are arranged parallel to one another along the curvature of the TLIF implant 100. As the inferior ridges 165 extend along their curvilinear path away from the insertion end 110, the inferior ridges.165 are interrupted to form a plurality of inferior teeth 167. The inferior teeth 167 are disposed at the engagement end 115 and on the anterior and posterior sides of the axial bore 140. The superior and inferior ridges 160, 165 guide the insertion of the TLIF implant 100 under the compressive forces of the adjacent vertebral bodies, while the superior and inferior teeth 162, 167 assist in the primary fixation of the TLIF implant 100.

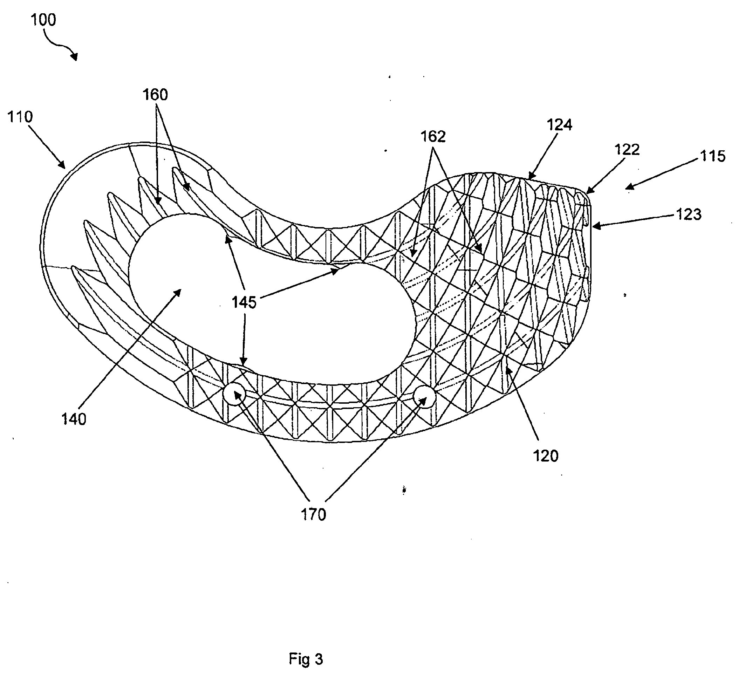

Referring to Figs. 3, 4, and 6, one or more radiopaque markers 170, made from material capable of radiographical imaging, such as pins or beads of stainless steel, titanium, tantalum, titanium-aluminum-niobium (TAN), or the like, are included in the TLIF implant 100 for enabling visualization and controlling of the position of the TLIF implant 100 during and after insertion into the disc space. In a first preferred embodiment, the markers 170 are elongated and include a first marker 170A, a second marker 170B, and a third marker 170C. The first and second markers 170A, 170B are disposed in the cranial-caudal direction on either side of the lateral window 150 within the anterior wall 130 of the implant 100. The third marker 170C is disposed proximate the insertion end 110, with a longitudinal axis thereof extending from the insertion end 110 toward the axial bore 140.

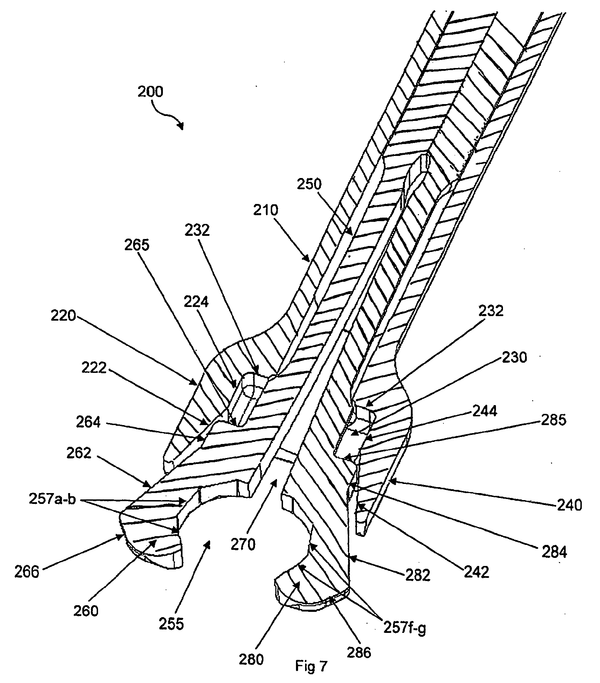

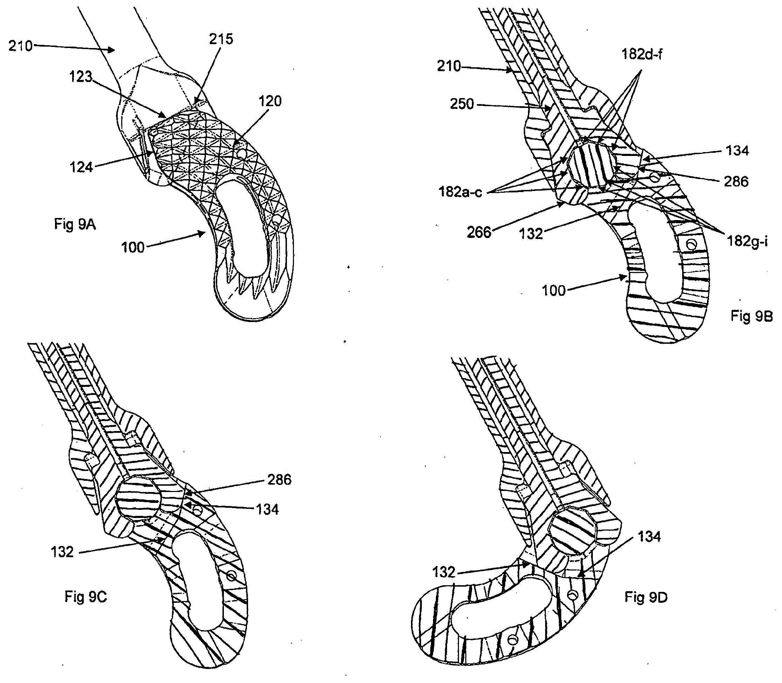

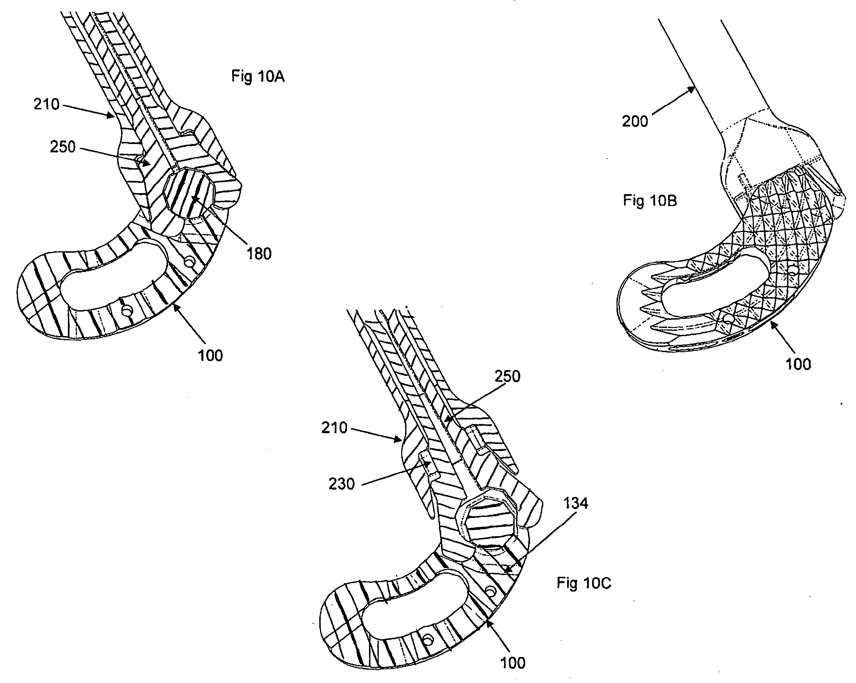

The engagement end 115 is characterized by the absence of the walls 130 extending fully between the superior and inferior surfaces 120, 125. That is, a slot 135 is formed at the engagement end 115 that extends continuously between and at least partially along the anterior and posterior walls 130. A post 180 is positioned within the slot 135, which is spaced apart from the anterior and posterior walls 130 and extends at least partially between the superior and inferior surfaces 120, 125 and serves as an instrument engagement feature. Adequate space is provided by the slot 135 for the engagement portion of an instrument 200 (Fig. 7) to engage the post 180. As shown in Figs. 9 and 10, within the implant 100, the walls 130 disposed between the axial bore 140 and the post 180 include first and second mating surfaces 132, 134 facing the post 180 between which an obtuse angle is formed for providing a pair of mechanical stops to the range of allowable articulation of the implant 100 with respect to the instrument 200. The first and second mating surfaces 132, 134 are preferably linear surfaces, but may also be curved or the like. Alternatively, stop pins or the like may be used to limit articulation of the implant 100.

Referring now to Figs. 9 and 10, in a first preferred embodiment, the post 180 is polygonal in cross-section and includes nine exposed facets 182a-182i arranged around an entire periphery thereof and extending in the cranial-caudal direction between the superior and inferior surfaces 120, 125. The facets 182a-182i are configured to enhance the engagement and interaction between the instrument 200 and the implant 100 during the insertion of the implant 100. Preferably, seven of the facets 182a-182f, 182i are flat surfaces, while the remaining two facets 182g-182h are curved surfaces. In an alternate embodiment, the post 180 may include a different polygonal number of facets 182. In yet another alternate embodiment, the post 180 can be cylindrical and thus include zero facets 182, and may include other features for governing the articulation of the implant 100 with respect to the instrument 200 during its insertion. For example, the post 180 can include dimples, teeth, surface texturing, grooves, or the like.

Referring now to Figs. 1-5 and 7, the engagement end 115 of the superior surface 120 terminates in a superior corner 122, which includes superior first and second flat segments 123, 124 originating near the post 180 and converging at an angle disposed proximate the engagement end 115 of the implant 100. Similarly, the engagement end 115 of the inferior surface 125 terminates in an inferior corner 127, which include inferior first and second flat segments 128, 129 originating near the post 180 and converging terminating at an angle disposed proximate the engagement end 115 of the implant 100. The superior first flat segment 123 and the inferior first flat segment 128 are configured to be engagable by a portion of the instrument 200, as is described in detail below, to provide a toggle-free connection, as are the superior second flat segment 124 and the inferior second flat segment 129. The rims of both the superior and inferior corner segments 122, 127 have a width extending a short distance from the superior and inferior surfaces 120, 125 toward the center of the implant 100. The surfaces of the rims are also flat for enhancing the interaction between the instrument 200 and the implant 100. The implant 100 can be formed from a variety of biocompatible materials, including but not limited to titanium, stainless steel, allograft bone, or polymers such as polyaryletheretherketone (PEEK) and polyetherketoneketone (PEKK), titanfoam, porous PEEK, or the like.

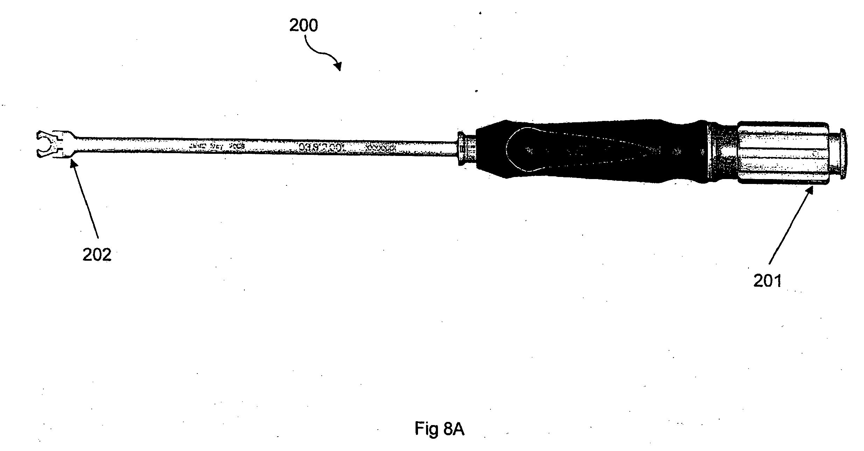

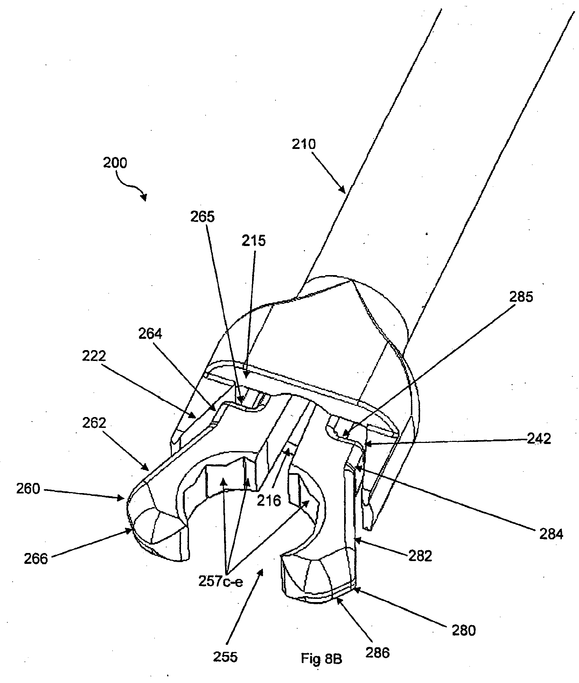

Referring to Figs. 7-8, an instrument 200 is provided that includes a longitudinal axis extending between a proximal end 201 and a distal end 202. The instrument 200 includes an elongated cannulated outer member 210 that surrounds an elongated inner member 250. The inner member 250 is configured to be translatable with respect to the outer member 210 along the longitudinal axis. Alternatively, the instrument 200 can be configured such that the outer member 210 is translatable with respect to the inner member 250 along the longitudinal axis to perform in the same manner. The proximal end of the outer member 210 includes a handle portion (not shown) and an actuation mechanism (not shown) for translating the inner member 250 with respect to the outer member 210. The distal end of the outer member 210 includes an outer member first arm 220 and an outer member second arm 240 that are separated by a gap 230 that forms the distal portion of the cannula. The gap 230 includes a pair of laterally-oriented surfaces 232 on either side of the cannula disposed at the proximal end of the outer member first and second arms 220, 240. The laterally-oriented surfaces 232 serve as a stop to the retraction of the inner member 250 with respect to the outer member 210. The interior surface of the first arm 220 includes an outer member first arm interior linear taper 222 disposed distal to an outer member first arm interior straight portion 224,while the interior surface of the second arm 240 includes an outer member second arm interior linear taper 242 disposed distal to an outer member second arm interior straight portion 244. The first and second arm interior linear tapers 222, 242 combine to form two wedging surfaces.

A laterally-extending superior exterior flat surface 215 of the outer member 210 is disposed between the distal ends of the outer member first and second arms 220, 240 and the laterally-oriented surfaces 232. Similarly, a laterally-extending inferior exterior flat surface 216 of the outer member 210 is disposed between the distal ends of the outer member first and second arms 220, 240 and the laterally-oriented surfaces 232. The laterally-extending superior exterior flat surface 215 and the laterally-extending inferior exterior flat surface 216 are configured to serve as stops to prevent over articulation of the implant 100 by abutting the superior and inferior first flat segments 123, 128 at one end of the articulation range and interacting with the superior and inferior second flat segments 124, 129 at the other end of the articulation range, as is described in detail below. The laterally-extending superior and inferior exterior flat surfaces 215, 216 also abut against the superior and inferior first flat segments 123, 128 of the implant 100, or against the superior and inferior second flat segments 124, 129 of the implant 100, during a portion of the implant insertion procedure.

The inner member 250 includes at its distal end a grasping portion 255 an inner member first arm 260 and an inner member second arm 280 separated by a split 270 that extends through the middle of the inner member 250 along the longitudinal axis from the grasping portion 255 toward the proximal end. The interior surface of the grasping portion 255 includes a plurality of engagement surfaces 257 that are configured to complementarily match the polygonal cross sectional geometry of the post 180 of the implant 100 and, thus, engage several of the plurality of facets 182a-182i. In a first preferred embodiment, there are seven engagement surfaces 257a-257g that are configured to engage seven of the nine facets 182a-182i of the post 180. Configured to interact with the interior surfaces of the outer member first and second arms 220, 240, the exterior surface of the inner member first arm 260 includes an inner member first arm exterior linear taper 262 disposed distal to an inner member first arm exterior straight portion 264, while the exterior surface of the inner member second arm 280 includes an inner member second arm exterior linear taper 282 disposed distal to an inner member second arm exterior straight portion 284. Disposed between the inner member first arm exterior linear taper 262 and the distal tip of the inner member first arm 260 is an inner member first arm second exterior linear taper 266.

Similarly, disposed between the inner member second arm exterior linear taper 282 and the distal tip of the inner member second arm 280 is an inner member second arm second exterior linear taper 286. Further, an inner member first arm laterally-oriented flat surface 265 and an inner member second arm laterally-oriented flat surface 285 are formed proximal to and adjacent the inner member first arm exterior straight potion 264 and the inner member second arm exterior straight portion 284, respectively, such that a pair of corners are formed therebetween, and such that the inner member first and second arm laterally-oriented flat surfaces 265, 285 face and abut with the laterally-oriented surfaces 232.

Referring to Fig. 12, a trial implant 300 is provided that includes geometry and surface features identical or similar to the implant 100 and further includes a lateral hole 310 and a longitudinal hole 320 and, therefore, a complete description of the trial implant is omitted for convenience only and is not limiting. The trial implant 300 is formed from a material that is visible under radiographic imaging, such as titanium, stainless steel, or the like. The lateral and longitudinal holes 310, 320, when viewed in conjunction with lateral and frontal X-rays, assist in the optimum positioning of the trial implant 300. The lateral holes 310 allow the surgeon to center the trial implant 300 with respect to the spinous processes of the vertebral bodies under fluoroscopy. The longitudinal hole 320 indicates whether the trial implant 300 has turned, in which case the surgeon will know that more disc material should preferably be removed. The lateral and longitudinal holes 310, 320 are shown as being generally circular or cylindrical in the preferred embodiment, but are not so limited. The lateral and longitudinal holes 310, 320 may have nearly any size and/or shape, such as rectangular, square, arrow-shaped, and/or triangular that permits visualization of the location of the trial implant 300 under imaging. In addition, the trial implant 300 is not limited to including the lateral and longitudinal holes 310, 320 or any holes, as location of the trial implant 300 may be visualized via markers or other features that are optically or machine viewable.

In operation, and in continuing reference to Figs. 1-12, a spinal disc in need of repair or replacement is identified and an at least partial discectomy is performed, preferably via a unilateral transforaminal approach. The trial implant 300 is inserted and removed using the instrument 200 to gauge the appropriate size implant 100 for insertion into the disc space. The insertion and manipulation of the trial implant 300 using the instrument 200 is identical to the method of inserting and manipulating the implant 100 using the instrument 200, as described below. The lateral and longitudinal holes 310, 320 are viewed using lateral and/or frontal X-rays to confirm the appropriate position of the trial implant 300 within the disc space and an implant size is then chosen.

Thus, the trial implant 300 is used for more than simply measuring the height between the vertebral bodies. Since the trial implant 300 articulates and is inserted to the same desired position as the final implant 100, the trial implant 300 may be used to determine whether the desired position of the implant 100 is reachable, whether enough disc material has been removed, and the like.

The bone graft 190 is then inserted into the axial bore 140 and secured therein via the surface features 145 (if not already preassembled thereto) and the implant 100 is then coupled to the instrument 200 by distracting the outer member 210 with respect to the inner member 250 via the manipulation of the actuation mechanism (not shown) such that the instrument 200 assumes an open configuration, as seen in Figs 7, 8, and 10C. The grasping portion 255 is then centered around the post 180 and the inner member 250 is partially retracted with respect to the outer member 210 via the manipulation of the actuation mechanism, thereby forcing the pair of corners formed between the inner member first and second arm exterior straight portions 264, 284 and the inner member first and second arm laterally-oriented flat surfaces 265, 285 to slidingly bear against the outer member first and second arm interior linear tapers 222, 242 until the inner member first and second arm exterior straight portions 264, 284 come to bear against the outer member first and second arm interior straight portions 224, 244, while providing the gap 230 between the inner member first and second arm laterally-oriented flat surfaces 265, 285 and the laterally-oriented surfaces 232. Consequently, the grasping portion 255 is collapsed around the post 180 such that the engagement surfaces 257a-g come into contact against the plurality of facets 182a-i of the post 180 and such that the post 180 is provisionally captured by the grasping portion 255, as shown in Fig. 9C, with the inner member first arm second exterior linear taper 286 bearing against the second linear surface 134.

In this provisionally locked configuration, the implant 100 is secured to the instrument but the post 180 is capable of rotation with respect to the grasping portion 255 but is prevented from exiting from the grasping portion 255. Final locking of the grasping portion 255 about the post 180, as shown in Figs. 9A, 9B, 10A, and 10B, is achieved by fully retracting the inner member 250 with respect to the outer member 210 via the continued manipulation of the actuation mechanism, thereby forcing the outer member first arm interior linear taper 222 and the outer member second arm interior linear taper 242 to come to bear against the inner member first arm exterior linear taper 262 and the inner member second arm exterior linear taper 282, respectively, thereby closing the gap 230, and finally locking the implant 100 to the instrument 200 while preventing any portions of the inner member first and second arms 260, 280 from separating under force from one another across the split 270 due to the contact between the outer member first and second arm interior linear tapers 222, 242 and the inner member first and second arm exterior linear tapers 262, 282. In this finally locked configuration, the superior and inferior exterior flat surfaces 215, 216 contact the superior and inferior first flat segments 123, 128, respectively, the gap 230 is closed, the inner member first arm second exterior linear taper 286 still bears against the second linear surface 134, and the post 180 is incapable of rotating with respect to the grasping portion 255.

In the finally locked configuration, the handle portion of the instrument 200 is grasped and the insertion end 110 of the implant is inserted into the transforaminal window created during the discectomy procedure until the bullet nose 112 enters the disc space and begins to distract the adjacent vertebral bodies and the distal end of the superior and inferior ridges 160, 165 make contact with the inferior surface of the superior vertebral body and the superior surface of the inferior vertebral body, respectively. Gentle hammer blows or other impaction forces are administered to the proximal end 201 of the instrument 200 to urge the implant 100 at least partially into the disc space. Toggling is prevented between the implant 100 and the instrument 200 during the delivery of impaction forces due to the abutment of (1) the superior and inferior first flat segments 123, 128 with the superior and inferior exterior flat surfaces 215, 216 and/or (2) the second linear surface 134 with the first arm second linear taper 286 and/or (3) the plurality of facets 182a-i of the post 180 with the engagement surface 257a-f when the instrument 200 is in its finally locked configuration with respect to the implant 100. Any of these abutments alone or in combination preferably prevent toggling between the implant 100 and the instrument 200 in the finally locked configuration.

Once the impaction forces drive the implant 100 along a linear path to a desired position within the disc space, as seen in Fig. 11A, with the instrument 200 finally locked to the implant 100, the inner member 250 is advanced with respect to the outer member 210 such that the instrument reassumes its provisionally locked configuration with respect to the implant 100, in which the implant 100 is coupled to the instrument but the post 180 is capable of rotation with respect to the grasping portion 255. At this point, additional gentle hammer blows or other impaction forces are administered to the proximal end of the instrument 200 and the superior and inferior ridges 160, 165 contact the endplates of the vertebral bodies to promote turning of the implant 100 and guide the path of insertion of the implant 100 as the insertion end 110 progresses into the disc space. As the superior and inferior ridges 160, 165 guide the implant 100 into the desired position within the disc space, the post 180 and, hence, the implant 100, rotates with respect to the grasping portion 255 within a range restricted by the stops provided by the interaction between the inner member second arm second exterior linear taper 286 bearing against the second linear surface 134 (the starting configuration of the insertion method) and the inner member first arm second exterior linear taper 266 bearing against the first linear surface 132 (at maximum angulation).

Throughout the entirety of the insertion process, the angle of the shaft of the instrument 200 with respect to the disc space is maintained constant, as all of the action performed to articulate the implant 100 is undertaken by the implant 100 itself as the gentle impaction forces drive the implant 100 into its desired final position guided by the superior and inferior ridges 160, 165, with no active turning of the implant necessary. Upon contact between the inner member first arm second exterior linear taper 266 and the first linear surface 132, the implant 100 is at or near its desired final positioning interior to the disc space. At this point, the implant 100 can be repositioned as necessary by again finally locking the implant 100 to the instrument 200, by retracting the inner member 250 distally with respect to the outer member 210, and manipulating the handle of the instrument 200 until the optimum final positioning of the implant 100 is achieved with respect to the disc space while viewing the position of the markers 170 under fluoroscopic imaging. The arrangement of the markers 170 enables a single radiographic image, e.g., a lateral image, to be used to determine the precise position of the implant 100 with respect to the disc space. The implant 100 is then released from the instrument 200 by manipulating the actuation mechanism until the instrument 200 assumes its open configuration, as described previously, and the grasping portion 255 no longer contacts the post 180. The compression forces between the vertebral endplates and the superior and inferior surfaces 120, 125 maintain the implant 100 in place as the instrument 200 is removed from the disc space and the patient's body.

The insertion and removal of the trial implant 300 may cause the formation of grooves in the adjacent endplates of the superior and inferior vertebral bodies due to the inclusion on the superior and inferior surfaces of the trial implant 300 of superior and inferior ridges that are identical to the superior and inferior ridges 160, 165 of the implant 100. The formation of such grooves in the adjacent endplates of the superior and inferior vertebral bodies, while not required for insertion of the implant 100, may assist in easing the insertion of the implant 100 using the instrument 200 via the guided mating of the superior and inferior ridges 160, 165 with the grooves formed previously by the trial implant 300.

While embodiments of the present invention are described herein with respect to an interbody spacer configured for insertion via a transforaminal path, a variety of implants may be utilized, such as total disc replacements and nucleus replacement devices, by simply configuring such implants to include an appropriately faceted post for an instrument engagement feature and, optionally, the stops and toggle-free bearing surfaces described herein. As such, the implant 100 is not limited to a banana or kidney bean shape, but may assume any geometry that can be accommodated within the disc space. Further, a range of angular approaches to the disc space may be utilized where an elongated implant is desired to be manipulated or pivoted once it has been delivered along a straight path into the disc space, such as posterior-lateral approaches, translateral, and direct lateral procedures.

In an alternate embodiment, the non-toggling interface between the implant 100 and the instrument 200 during the delivery of impaction forces that is provided by the interaction and abutment of the superior and inferior first flat segments 123,128 with the laterally extending superior and inferior exterior flat surfaces 215, 216, as well as the interaction and abutment of the superior and inferior second flat segments 124, 129 with the laterally-extending superior and inferior exterior flat surfaces 215, 216, can also be provided with nonlinear abutment surfaces. As long as the surfaces mate or are able to abut one another when the instrument assumes its finally locked configuration, a non-toggling interface can be provided.

Similarly, the articulation stops that prevent over articulation of the implant 100 with respect to the instrument 200 that are embodied by the first and second linear surfaces 132, 134, and the range of articulation provided by the obtuse angle disposed therebetween, can be provided by a variety of angles which can be tailored specifically to a desired articulation range for a given application, and therefore does not necessarily need to be obtuse. Further, the first and second linear surfaces 132, 134, as well as the inner member first and second arm second exterior linear tapers 266, 286 that are abutted thereagainst, need not be linear surfaces. Rather, any mating abutment surfaces will suffice between 132 and 266 and between 134 and 286 for the purposes of limiting the articulation range. Further, an embodiment may be envisioned in which the obtuse angle is removed between the first and second linear surfaces 132, 134 such that a single abutment surface is provided that can limit the range of articulation by being abuttable by both the first and second arm second exterior linear tapers 266, 286 and, further, does not need to be linear as long as it provides a mating abutment surface to the geometry chosen for the first and second arm second exterior linear tapers 266, 286.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the present description.

## Claims

1. A system for placing an intervertebral implant in a disc space disposed between first and second vertebral endplates of adjacent vertebral bodies of a patient, the system comprising:
(a) an intervertebral implant (100), comprising:
(i) an insertion end (110) and an opposing engagement end (115);
(ii) first and second opposed main surfaces (120, 125) configured to contact the respective adjacent first and second vertebral endplates, each of the first and second main surfaces (120, 125) having an anterior edge, and a posterior edge opposed to the anterior edge, the anterior and posterior edges extending between the insertion and engagement ends (110, 115);
(iii) an anterior wall (130) formed between the first and second main surfaces (120, 125) and disposed along the anterior edges thereof;
(iv) a posterior wall (130) formed between the first and second main surfaces (120, 125) and disposed along the posterior edges thereof, the anterior wall (130) and the posterior wall (130) converging at the insertion end and the engagement end (110, 115);
(v) a slot (135) formed at the engagement end (115) and extending continuously between and at least partially along the anterior and posterior walls (130); and
(vi) a post (180) positioned within the slot (135), the post spaced from at least one of the anterior and posterior walls (130) and extending at least partially between the first and second main surfaces (120, 125), the post (180) having a plurality of exposed facets, and
(b) an insertion instrument (200) including:
(i) a proximal end and a distal end spaced from the proximal end along a longitudinal axis,
(ii) an inner member (250) and an outer member (210), the inner member (250) being translatable with respect to the outer member (210) along the longitudinal axis, the inner member having a grasping portion (255) disposed at the distal end of the instrument, the grasping portion (255) including a plurality of engagement surfaces (257) that are configured to complementarily match the plurality of exposed facets of the post (180) of the intervertebral implant (100);
(b) the instrument (200) configured to engage the post (180) of the intervertebral implant (100), the instrument having 1) a first configuration in which the grasping portion (255) is open for coupling to the post (180) of the intervertebral implant (100), 2) a second configuration in which the instrument (200) is securely coupled to the post (180) of the intervertebral implant (100) while allowing the post (180) of the intervertebral implant (100) to rotate with respect to the grasping portion (255) upon application of an impaction force to the instrument, and 3) a third configuration in which the instrument (200) is securely coupled to the post (180) of the intervertebral implant (100) so as to prevent rotation of the post (180) with respect to the grasping portion (255).

2. The system of claim 1, wherein each anterior edge has a generally linear portion (124, 129) proximate the engagement end (115) and a generally concave portion, and each posterior edge has a generally linear portion (123, 128) proximate the engagement end (115) and a generally convex portion, the generally linear portion (124, 129) of the anterior edge converging with the generally linear portion (123, 128) of the posterior edge at the engagement end (115) for each of the first and second main surfaces (120, 125).

3. The system of claim 1 or 2, wherein each anterior edge is generally concave and each posterior edge is generally convex, and the post (180) includes a plurality of exposed facets (182a-182i) that are disposed around an entire periphery of the post (180), wherein the intervertebral implant includes at least one abutment surface (132, 134) that is disposed within the slot (135) from the post (180) in a distal direction that extends from the engagement end toward the insertion end, the at least one abutment surface (132, 134) limiting rotation of the implant (100) about the post (180) when the post (180) is engaged with the instrument (200).

4. The system of any one of claims 1 to 3, wherein the outer member (210) is formed as an elongated cannulated outer member (210) and the inner member (250) is formed as an elongated inner member (250), wherein the elongated cannulated outer member (210) surrounds the elongated inner member (250).

5. The system of claim 4, wherein a proximal end of the outer member (210) includes a handle portion and an actuation mechanism for translating the inner member (250) with respect to the outer member (210).

6. The system of claim 5, wherein the actuation mechanism is configured to cause the inner member (250) to translate relative to the outer member (210) such that the grasping portion transitions between the second configuration and the third configuration.

7. The system of claim 6, wherein when the instrument (200) is in the second configuration, the grasping portion at least partially surrounds the post (180) but it is disengaged from the post (180) so that the grasping portion is rotatable with respect to the post (180), and/or wherein when the instrument (200) is in the third configuration, the grasping portion at least partially surrounds the post (180) and is engaged with the post (180) so that the instrument (200) is rotationally fixed to the post (180).

8. The system of any one of claims 4 to 7, wherein the distal end of the outer member (210) includes an outer member first arm (220) and an outer member second arm (240) that are separated by a gap (230).

9. The system of claim 8, wherein the gap (230) includes a pair of laterally-oriented surfaces (232) on either side of the outer member (210) disposed at a proximal end of the outer member first and second arms (220, 240), wherein the laterally-oriented surfaces (232) serve as a stop to a retraction of the inner member (250) with respect to the outer member (210).

10. The system of claim 8 or 9, wherein an interior surface of the first arm (220) includes an outer member first arm interior linear taper (222) disposed distal to an outer member first arm interior straight portion (224), while an interior surface of the second arm (240) includes an outer member second arm interior linear taper (242) disposed distal to an outer member second arm interior straight portion (244), wherein the first and second arm interior linear tapers (222, 242) combine to form two wedging surfaces.

11. The system of any one of claims 4 to 10, with claims 2 and 9, wherein a laterally-extending superior exterior flat surface (215) of the outer member (210) is disposed between distal ends of the outer member first and second arms (220, 240) and the laterally-oriented surfaces (232), and a laterally-extending inferior exterior flat surface (216) of the outer member (210) is disposed between the distal ends of the outer member first and second arms (220, 240) and the laterally-oriented surfaces (232).

12. The system of claim 11, wherein the laterally-extending superior exterior flat surface (215) and the laterally-extending inferior exterior flat surface (216) are configured to serve as stops to prevent over articulation of the implant (100) by abutting the superior and inferior linear portions (123, 28) of the posterior edges at one end of the articulation range and interacting with the superior and inferior linear portions (124, 129) of the anterior portions at the other end of the articulation range.

13. The system of any one of claims 4 to 12, wherein the inner member (250) includes at its distal end an inner member first arm (260) and an inner member second arm (280) separated by a split (270) that extends through the middle of the inner member (250) along the longitudinal axis from the grasping portion (255) toward the proximal end of the instrument.

14. The system of any one of claims 1 to 13, wherein the delivery of impaction forces to the instrument when the grasping portion is in the third configuration causes the intervertebral implant to advance in the disc space.

15. The system of any one of claims 1 to 14, wherein the delivery of impaction forces to the instrument (200) when the grasping portion is in the second configuration causes the intervertebral implant (100) to rotate with respect to the instrument (200) until a portion of the instrument abuts the at least one abutment surface.

## Patentansprüche

1. System zum Platzieren eines Zwischenwirbelimplantats in einem Scheibenraum, der sich zwischen einer ersten und zweiten Endplatte von benachbarten Wirbelkörpern eines Patienten befindet, wobei das System umfasst:
(a) ein Zwischenwirbelimplantat (100), umfassend:
(i) ein Einführende (110) und ein gegenüberliegendes Eingriffsende (115),
(ii) eine erste und zweite gegenüberliegende Hauptoberfläche (120,125), die eingerichtet sind, die entsprechenden benachbarten ersten und zweiten Wirbelkörperendplatten zu kontaktieren, wobei jede der ersten und zweiten Hauptoberfläche (120,125) einen anterioren Rand und einen posterioren Rand gegenüber dem anterioren Rand aufweist, wobei sich der anteriore Rand und der posteriore Rand zwischen dem Einführ- und Eingriffsende (110, 115) erstrecken,
(iii) eine anteriore Wand (130), die zwischen der ersten und zweiten Hauptoberfläche (120, 125) gebildet und entlang deren anterioren Rändern angeordnet ist,
(iv) eine posteriore Wand (130), die zwischen der ersten und zweiten Hauptoberfläche (120, 125) gebildet und entlang deren posterioren Rändern angeordnet ist, wobei die anteriore Wand (130) und die posteriore Wand (130) an dem Einführende und dem Eingriffsende (110,115) konvergieren,
(v) einen Schlitz (135), der an dem Eingriffsende (115) gebildet ist und sich kontinuierlich zwischen und zumindest teilweise entlang der anterioren und posterioren Wand (130) erstreckt, und
(vi) eine Säule (180), die in dem Schlitz (135) positioniert ist, wobei die Säule von zumindest einer der anterioren und posterioren Wand (130) beabstandet ist und sich zumindest teilweise zwischen der ersten und zweiten Hauptoberfläche (120, 125) erstreckt, wobei die Säule (180) eine Mehrzahl von freiliegenden Facetten aufweist, und
(b) ein Einführinstrument (200), enthaltend:
(i) ein proximales Ende und ein von dem proximalen Ende entlang einer Längsachse beabstandetes distales Ende,
(ii) ein inneres Element (250) und ein äußeres Element (210), wobei das innere Element (250) bezüglich des äußeren Elements (210) entlang der Längsachse verschieblich ist, wobei das innere Element einen Greifteil (255) angeordnet an dem distalen Ende des Instruments aufweist, wobei der Greifteil (255) eine Mehrzahl von Eingriffsoberflächen (257) enthält, die eingerichtet sind, mit einer Mehrzahl von freiliegenden Facetten der Säule (180) des Zwischenwirbelimplantats (100) komplementär zusammenzupassen,
wobei das Instrument (200) eingerichtet ist, mit der Säule (180) des Zwischenwirbelimplantats (100) zusammenzuwirken, wobei das Instrument 1) eine erste Konfiguration aufweist, in welcher der Greifteil (255) zum Koppeln mit der Säule (180) des Zwischenwirbelimplantats (100) offen ist, 2) eine zweite Konfiguration, in welcher das Instrument (200) sicher mit der Säule (180) des Zwischenwirbelimplantats (100) gekoppelt ist, während es der Säule (180) des Zwischenwirbelimplantats (100) erlaubt ist, in dem Greif teil (255) unter Anwendung einer Einwirkkraft auf das Instrument zu drehen, und 3) eine dritte Konfiguration, in welcher das Instrument (200) sicher mit der Säule (180) des Zwischenwirbelimplantats (100) gekoppelt ist, um eine Drehung der Säule (180) bezüglich des Greifteils (255) zu verhindern.

2. System nach Anspruch 1, wobei jeder anteriore Rand einen im Allgemeinen linearen Teil (124,129) nahe dem Eingriffsende (115) und einen im Allgemeinen konkaven Teil aufweist und jeder posteriore Rand einen im Allgemeinen linearen Teil (123, 128) nahe dem Eingriffsende (115) und einen im Allgemeinen konvexen Teil aufweist, wobei der im Allgemeinen lineare Teil (124,129) des anterioren Rands mit dem im Allgemeinen linearen Teil (123, 128) des posterioren Rands an dem Eingriffsende (115) für jede der ersten und zweiten Hauptoberfläche (120,125) konvergiert.

3. System nach Anspruch 1 oder 2, wobei jeder anteriore Rand im Allgemeinen konkav ist und jeder posteriore Rand im Allgemeinen konvex ist, und die Säule (180) eine Mehrzahl von freiliegenden Facetten (182a - 182i) aufweist, die um einen gesamten Umfang der Säule (180) herum angeordnet sind, wobei das Zwischenwirbelimplantat zumindest eine Anlageoberfläche (132, 134) enthält, die in dem Schlitz (135) von der Säule (180) in einer distalen Richtung angeordnet ist, die sich von dem Eingriffsende zu dem Einführende erstreckt, wobei die zumindest eine Anlageoberfläche (132, 134) eine Drehung des Implantats (100) um die Säule (180) herum begrenzt, wenn die Säule (180) mit dem Instrument (200) in Eingriff ist.

4. System nach einem der Ansprüche 1 bis 3, wobei das äußere Element (210) als ein längliches kanüliertes äußeres Element (210) ausgebildet ist und das innere Element (250) als ein längliches inneres Element (250) ausgebildet ist, wobei das längliche kanülierte äußere Element (210) das längliche innere Element (250) umgibt.

5. System nach Anspruch 4, wobei ein proximales Ende des äußeren Elements (210) einen Griffteil und einen Betätigungsmechanismus zum Verschieben des inneren Elements (250) bezüglich des äußeren Elements (210) enthält.

6. System nach Anspruch 5, wobei der Betätigungsmechanismus eingerichtet ist, zu bewirken, dass sich das innere Element (250) relativ zu dem äußeren Element (210) verschiebt, so dass der Greifteil zwischen der zweiten Konfiguration und der dritten Konfiguration übergeht.

7. System nach Anspruch 6, wobei, wenn das Instrument (200) in der zweiten Konfiguration ist, der Greifteil zumindest teilweise die Säule (180) umgibt, aber von der Säule (180) gelöst ist, so dass der Greifteil bezüglich der Säule (180) drehbar ist, und/oder wobei, wenn das Instrument (200) in der dritten Konfiguration ist, der Greifteil zumindest teilweise die Säule (180) umgibt und mit der Säule (180) in Eingriff ist, so dass das Instrument (200) an der Säule bezüglich Drehung fixiert ist.

8. System nach einem der Ansprüche 4 bis 7, wobei das distale Ende des äußeren Elements (210) einen ersten Arm (220) des äußeren Elements und einen zweiten Arm (240) des äußeren Elements enthält, die durch eine Lücke (230) getrennt sind.

9. System nach Anspruch 8, wobei die Lücke (230) ein Paar von seitlich orientierten Oberflächen (232) auf jeder Seite des äußeren Elements (210) enthält, die an einem proximalen Ende des ersten und zweiten Arms (220, 240) des äußeren Elements angeordnet sind, wobei die seitlich orientierten Oberflächen (232) als ein Anschlag für ein Zurückziehen des inneren Elements (250) bezüglich des äußeren Elements (210) dienen.

10. System nach Anspruch 8 oder 9, wobei eine innen liegende Oberfläche des ersten Arms (220) eine innen liegende lineare Abschrägung (222) des ersten Arms des äußeren Elements enthält, die distal zu einem innen liegenden geraden Teil (224) des ersten Arms des äußeren Elements angeordnet ist, während eine innen liegende Oberfläche des zweiten Arms (240) eine innen liegende lineare Abschrägung (242) des zweiten Arms des äußeren Elements enthält, die distal zu einem innen liegenden geraden Teil (244) des zweiten Arms des äußeren Elements angeordnet ist, wobei die innen liegenden linearen Abschrägungen (222, 242) des ersten und zweiten Arms kombiniert sind, um zwei Keiloberflächen zu bilden.

11. System nach einem der Ansprüche 4 bis 10, mit den Ansprüchen 2 und 9, wobei eine sich seitlich erstreckende obere außen liegende flache Oberfläche (215) des äußeren Elements (210) zwischen distalen Enden des ersten und zweiten Arms (220, 240) des äußeren Elements und den seitlich orientierten Oberflächen (232) angeordnet ist, und eine sich seitlich erstreckende untere außen liegende flache Oberfläche (216) des äußeren Elements (210) zwischen distalen Enden des ersten und zweiten Arms (220, 240) des äußeren Elements und den seitlich orientierten Oberflächen (232) angeordnet ist.

12. System nach Anspruch 11, wobei die sich seitlich erstreckende obere außen liegende flache Oberfläche (215) und die sich seitlich erstreckende untere außen liegende flache Oberfläche (216) eingerichtet sind, als Anschläge zu dienen, um eine Über-Abwinkelung des Implantats (100) zu verhindern, durch Anliegen des oberen und unteren linearen Teils (123, 28) der posterioren Ränder an einem Ende des Abwinkelungsbereichs und Interagieren mit dem oberen und unteren linearen Teil (124, 129) der anterioren Teile am anderen Ende des Abwinkelungsbereichs.

13. System nach einem der Ansprüche 4 bis 12, wobei das innere Element (250) an seinem distalen Ende einen ersten Arm (260) des inneren Elements und einen zweiten Arm (280) des inneren Elements enthält, die durch einen Spalt (270) getrennt sind, der sich durch die Mitte des inneren Elements (250) entlang der Längsachse von dem Greifteil (255) in Richtung des proximalen Endes des Instruments erstreckt.

14. System nach einem der Ansprüche 1 bis 13, wobei das Zuführen von Einwirkkräften auf das Instrument, wenn der Greifteil in der dritten Konfiguration ist, bewirkt, dass das Zwischenwirbelimplantat in den Scheibenraum vorgeschoben wird.

15. System nach einem der Ansprüche 1 bis 14, wobei das Zuführen von Einwirkkräften auf das Instrument (200), wenn der Greifteil in der zweiten Konfiguration ist, bewirkt, dass sich das Zwischenwirbelimplantat (100) bezüglich des Instruments (200) dreht, bis ein Teil des Instruments an der zumindest einen Anlageoberfläche anliegt.

## Revendications

1. Système destiné à placer un implant intervertébral dans un espace de disque disposé entre des premier et second plateaux vertébraux de corps vertébraux adjacents d'un patient, le système comprenant :
(a) un implant intervertébral (100), comprenant :
(i) une extrémité d'introduction (110) et une extrémité de prise opposée (115) ;
(ii) des première et seconde surfaces principales opposées (120, 125) configurées pour être en contact avec les premier et second plateaux vertébraux adjacents respectifs, chacune des première et seconde surfaces principales (120, 125) présentant un bord antérieur et un bord postérieur opposé au bord antérieur, les bords antérieur et postérieur s'étendant entre les extrémités d'introduction et de prise (110, 115) ;
(iii) une paroi antérieure (130) formée entre les première et seconde surfaces principales (120, 125) et disposée le long des bords antérieurs de celles-ci ;
(iv) une paroi postérieure (130) formée entre les première et seconde surfaces principales (120, 125) et disposée le long des bords postérieurs de celles-ci, la paroi antérieure (130) et la paroi postérieure (130) convergeant au niveau de l'extrémité d'introduction et de l'extrémité de prise (110, 115) ;
(v) une fente (135) formée au niveau de l'extrémité de prise (115) et s'étendant en continu entre les parois antérieure et postérieure (130), et au moins partiellement le long de celles-ci, et
(vi) un tenon (180) positionné dans la fente (135), le tenon étant espacé d'au moins une des parois parmi les parois antérieure et postérieure (130) et s'étendant au moins partiellement entre les première et seconde surfaces principales (120, 125), le tenon (180) présentant une pluralité de facettes exposées, et
(b) un instrument d'introduction (200) incluant :
(i) une extrémité proximale et une extrémité distale espacée de l'extrémité proximale le long d'un axe longitudinal, et
(ii) un élément intérieur (250) et un élément extérieur (210), l'élément intérieur (250) pouvant effectuer une translation par rapport à l'élément extérieur (210) le long de l'axe longitudinal, l'élément intérieur présentant une partie de préhension (255) disposée au niveau de l'extrémité distale de l'instrument, la partie de préhension (255) incluant une pluralité de surfaces de prise (257) qui sont configurées pour coïncider de manière complémentaire avec la pluralité de facettes exposées du tenon (180) de l'implant intervertébral (100) ;
(b) l'instrument (200) étant configuré pour venir en prise avec le tenon (180) de l'implant intervertébral (100), l'instrument présentant 1) une première configuration dans laquelle la partie de préhension (255) est ouverte pour s'accoupler avec le tenon (180) de l'implant intervertébral (100) ; 2) une deuxième configuration dans laquelle l'instrument (200) est accouplé de manière ferme au tenon (180) de l'implant intervertébral (100) tout en permettant au tenon (180) de l'implant intervertébral (100) de tourner par rapport à la partie de préhension (255) suite à l'application d'une force d'impaction sur l'instrument, et 3) une troisième configuration dans laquelle l'instrument (200) est accouplé de manière ferme au tenon (180) de l'implant intervertébral (100) de manière à empêcher la rotation du tenon (180) par rapport à la partie de préhension (255).

2. Système selon la revendication 1, dans lequel chaque bord antérieur présente une partie généralement linéaire (124, 129) à proximité de l'extrémité de prise (115) et une partie généralement concave, et chaque bord postérieur présente une partie généralement linéaire (123, 128) à proximité de l'extrémité de prise (115) et une partie généralement convexe, la partie généralement linéaire (124, 129) du bord antérieur convergeant avec la partie généralement linéaire (123, 128) du bord postérieur au niveau de l'extrémité de prise (115) pour chacune des première et seconde surfaces principales (120, 125).

3. Système selon la revendication 1 ou 2, dans lequel chaque bord antérieur est généralement concave et chaque bord postérieur est généralement convexe, et le tenon (180) inclut une pluralité de facettes exposées (182a-182i) disposées autour d'une périphérie complète du tenon (180), dans lequel l'implant intervertébral inclut au moins une surface d'about (132, 134) disposée dans la fente (135) à partir du tenon (180) dans une direction distale qui s'étend de l'extrémité de prise vers l'extrémité d'introduction, la au moins une surface d'about (132, 134) limitant la rotation de l'implant (100) autour du tenon (180) lorsque le tenon (180) est en prise avec l'instrument (200).

4. Système selon l'une quelconque des revendications 1 à 3, dans lequel l'élément extérieur (210) se présente sous la forme d'un élément extérieur tubulaire allongé (210) et l'élément intérieur (250) se présente sous la forme d'un élément intérieur allongé (250), dans lequel l'élément extérieur tubulaire allongé (210) entoure l'élément intérieur allongé (250).

5. Système selon la revendication 4, dans lequel une extrémité proximale de l'élément extérieur (210) inclut une partie de poignée et un mécanisme d'actionnement destiné à la translation de l'élément intérieur (250) par rapport à l'élément extérieur (210).

6. Système selon la revendication 5, dans lequel le mécanisme d'actionnement est configuré pour amener l'élément intérieur (250) à effectuer une translation par rapport à l'élément extérieur (210), de sorte que la partie de préhension transite entre la deuxième configuration et la troisième configuration.

7. Système selon la revendication 6, dans lequel l'instrument (200) se trouve dans la deuxième configuration, la partie de préhension entoure au moins partiellement le tenon (180) mais est désolidarisée du tenon (180), de sorte que la partie de préhension peut tourner par rapport au tenon (180), et/ou dans lequel lorsque l'instrument (200) se trouve dans la troisième configuration, la partie de préhension entoure au moins partiellement le tenon (180) et est en prise avec le tenon (180), de sorte que l'instrument est fixé par rotation au tenon (180).

8. Système selon l'une quelconque des revendications 4 à 7, dans lequel l'extrémité distale de l'élément extérieur (210) inclut un premier bras d'élément extérieur (220) et un second bras d'élément extérieur (240), lesquels sont séparés par un espace (230).

9. Système selon la revendication 8, dans lequel l'espace (230) inclut une paire de surfaces d'orientation latérale (232) de chaque côté de l'élément extérieur (210) disposées au niveau d'une extrémité proximale des premier et second bras d'élément extérieur (220, 240), dans lequel les surfaces d'orientation latérale (232) servent de butée à une rétraction de l'élément intérieur (250) par rapport à l'élément extérieur (210).

10. Système selon la revendication 8 ou 9, dans lequel une surface intérieure du premier bras (220) inclut un amincissement intérieur de premier bras d'élément extérieur (222), disposé de manière distale par rapport à la partie droite intérieure de premier bras d'élément extérieur (224), tandis qu'une surface intérieure du second bras (240) inclut un amincissement linéaire intérieur de second bras d'élément extérieur (242), disposé de manière distale par rapport à une partie droite intérieure de second bras d'élément extérieur (244), dans lequel les premier et second amincissements linéaires intérieurs de premier et second bras (222, 242) se combinent pour former deux surfaces de calage.

11. Système selon l'une quelconque des revendications 4 à 10, conjointement aux revendications 2 et 9, dans lequel une surface plate extérieure supérieure s'étendant latéralement (215) de l'élément extérieur (210) est disposée entre des extrémités distales des premier et second bras d'élément extérieur (220, 240) et des surfaces d'orientation latérale (232), et une surface plate extérieure inférieure s'étendant latéralement (216) de l'élément extérieur (210) est disposée entre les extrémités distales des premier et second bras d'élément extérieur (220, 240) et les surfaces d'orientation latérale (232).

12. Système selon la revendication 11, dans lequel la surface plate extérieure supérieure s'étendant latéralement (215) et la surface plate extérieure inférieure s'étendant latéralement (216) sont configurées pour servir de butées et empêcher une articulation excessive de l'implant (100) en venant abuter avec les parties linéaires supérieure et inférieure (123, 28) des bords postérieurs au niveau d'une extrémité de la plage d'articulation et en créant une interaction avec les parties linéaires supérieure et inférieure (124, 129) des parties antérieures au niveau de l'autre extrémité de la plage d'articulation.

13. Système selon l'une quelconque des revendications 4 à 12, dans lequel l'élément intérieur (250) inclut, au niveau de son extrémité distale, un premier bras d'élément intérieur (260) et un second bras d'élément intérieur (280), séparés par une division (270) qui s'étend à travers le milieu de l'élément intérieur (250) le long de l'axe longitudinal de la partie de préhension (255) vers l'extrémité proximale de l'instrument.

14. Système selon l'une quelconque des revendications 1 à 13, dans lequel lorsque la partie de préhension se trouve dans la troisième configuration, l'application de forces d'impaction à l'instrument entraîne une progression de l'implant intervertébral dans l'espace de disque.

15. Système selon l'une quelconque des revendications 1 à 14, dans lequel lorsque la partie de préhension se trouve dans la deuxième configuration, l'application de forces d'impaction à l'instrument (200) entraîne une rotation de l'implant intervertébral (100) par rapport à l'instrument (200) jusqu'à ce qu'une partie de l'instrument vienne abouter avec la au moins une surface d'about.
